# EUROPEAN PATENT APPLICATION

(11) **EP 4 790 410 A1**
(43) Date of publication of application: **12.08.2026**
(21) Application number: 25156057.9
(22) Date of filing: 05.02.2025
(51) Int. Cl.: G01N 33/00, G01M 3/00, G01N 25/22

(54) **DETECTION DEVICE AND METHOD FOR DETECTING HYDROGEN**

(71) Applicant: AVL List GmbH, 8020 Graz (AT); Greenlight Innovation, Burnaby BC V5A 4W2 (CA)
(72) Inventor: KANAFANI, Fadi, Richmond, V7C 5S6 (CA)
(74) Representative: Gamper, Bettina

(57) **Abstract**

The present invention relates to a detection device (10) and a corresponding method for detecting hydrogen from a gas volume (Vg). The technique employs a hydrogen oxidizing catalytic converter material (11) arranged exposed to the gas volume (Vg). According to the invention, a temperature (Tcc) of the catalytic converter material (11) is detected and compared with at least one predetermined exothermic temperature (Texo) associated with a thermal impact of exothermic heat from oxidation reaction activities at the catalytic converter material (11). Depending on a determination of the comparison result, a hydrogen detection result is generated for indicating different available domains of detection result including a presence, a leakage intensity, a quantity or a concentration of hydrogen in the gas volume (Vg).

## Description

The present application relates to a method for detecting hydrogen from a gas volume, a detection device for detecting hydrogen from a gas volume, a fuel cell system comprising the detection device, an electrolyser cell system comprising the detection device and a test station for testing electrochemical cell systems comprising the detection device.

The method and the detection device according to the invention are suitable to be applied or integrated for surveillance of operational safety within a system or in an environment of a system consuming, producing, or otherwise using hydrogen containing gases.

In the state of art, it is known to deploy safety measures for avoiding the risk of an accumulation of explosive gas in technical objects or facilities, in particular if gases of higher hydrogen concentrations are involved, such as a fuel gas for fuel cells or a product gas from an electrolyser cell.

Known safety measures for electrochemical cells processing hydrogen include installation of intrinsically safe electrical equipment which means that all kinds of electrical components must be certified for not creating any sparks. Depending on the electrical components needed for all kinds of functionalities, this safety requirement can cause high costs.

Another safety measure is to provide explosion-proof components like a containment and piping which is also an expensive way to avoid potential impacts of a malfunction, however without even reducing any probability of explosion or other safety events.

Hydrogen detecting sensors are a known technique for detecting critical concentrations of hydrogen in air or other gases and can be combined with an alarm function or a shutdown function. However, present hydrogen sensors are still expensive and have shown a relatively slow response of sensitivity.

Further safety measures include catalyzation techniques for eliminating residual hydrogen in an exhaust gas stream of a system. Such catalyzation works effective for prevention of explosive hydrogen accumulation, however, due to a passive and silent operation in an exhaust stream, a catalyzation technique will not share evidence or a quantity of converted hydrogen and will not output a desired notification if a safety critical malfunction like a hydrogen leakage occurs during operation.

It is an object of the invention to provide a technique avoiding the before mentioned draw backs in the state of art. More particular, it is an object of the invention to provide a technique that enables a less complex, a cheaper, a safer, a quicker responding or otherwise improved detection of hydrogen, in particular, detection of a hydrogen leakage, preferably at or within an electrochemical energy conversion cell system, for securing a safe operation.

The object is solved by a method having the steps of claim 1 and a corresponding device having the features of claim 7. Further features and details of the invention result from the dependent claims, the specification, and the drawings. Features and details described in connection with the claimed method according to the invention naturally also apply in connection with the claimed detection device and the claimed systems according to the invention, and vice versa, so that the individual aspects of the invention are or can always be mutually referred to in the disclosure.

The invention proposes a method for detecting hydrogen from a gas volume, including an exposure of a hydrogen oxidizing catalytic converter material to the gas volume. According to the invention the method comprises the steps of
- detecting a temperature of the catalytic converter material;
- comparing the detected temperature with at least one predetermined exothermic temperature associated with a thermal impact of exothermic heat from oxidation reaction activities at the catalytic converter material; and
- generating a hydrogen detection result indicating at least a presence of hydrogen in the gas volume, if the detected temperature is equal or higher than the at least one exothermic temperature.

Moreover, the invention analogously proposes a detection device for detecting hydrogen from a gas volume, including an exposure of a hydrogen oxidizing catalytic converter material to the gas volume. According to the invention the detection device comprises a temperature sensor arranged to detect a temperature of the catalytic converter material, and a temperature monitoring unit for monitoring the detected temperature. For this purpose, the temperature monitoring unit includes the functional modules of a comparator module and a generating module. The comparator module is configured to compare the detected temperature with at least one predetermined exothermic temperature associated with a thermal impact of exothermic heat from oxidation reaction activities at the catalytic converter material. The generating module is configured to generate a hydrogen detection result indicating at least a presence of hydrogen in the gas volume, if the detected temperature is equal or higher than the at least one exothermic temperature.

Hence, for the first time, the invention provides a combined use of a catalytic converter not only for elimination, but also for detection and even quantitative measurement of eliminated hydrogen. The catalytic converter material has the property of oxidizing hydrogen to harmless product water by activating an exothermic oxidation reaction of the hydrogen. By thermally monitoring an amount of exothermic oxidation reaction activity at the catalytic converter, the invention also provides to simultaneously derive a general presence detection or a quantitative detection of hydrogen in a gas volume. The invention is based on the insight, that a measurement of temperature at the catalytic converter or in a gas flow downstream near the catalytic converter that is caused by exothermic oxidation reaction of hydrogen shows a strong, essentially linear correlation with a converted quantity of hydrogen and thus a correlation with an expected average hydrogen quantity, an expected hydrogen concentration, or an expected leakage rate of leaking hydrogen in a given volume capacity or a given flow rate of a gas volume.

As a first advantage, as mentioned before, in terms of a detection task compared to a known hydrogen sensor, the technique according to the invention does not only detect hydrogen but does also simultaneously eliminate the detected hydrogen. In this way the technique of the invention does not only provide information on presence or quantitative measurement, but provides right from the start of operation, the freedom of intrinsically safe operation due to its functionality of chemically disenabling the very first cause of explosive potential. The latter task of elimination is at least fulfilled if the catalytic converter material is sufficiently dimensioned to cope with a leakage scenario or other hydrogen involving malfunctions. Hence, due to the functionality of elimination in addition to the functionality of detection, a sole catalytic converter originally installed in a present system as well as an optional installation of a sole catalytic converter in future systems, employed as safety measure for avoiding explosive hydrogen accumulation, can be omitted while costs, number of parts and complexity are reduced.

A second advantage is based on a comparison to most known hydrogen sensors, wherein the technique according to the invention achieves a faster response of sensitivity.

Furthermore, a third advantage is to be found in a less complex and more robust design compared to most known hydrogen sensors leading to an expected cost advantage and an expected longer life span.

According to an advantageous aspect of the invention, the method comprises the steps of:
- determining a value of leakage intensity or a value of hydrogen quantity of hydrogen in the gas volume based on a predetermined relation or on predetermined correlated values between at least one of the detected temperatures and a detected gas pressure (Pg), and a corresponding leakage intensity or a corresponding hydrogen quantity; and
- generating a hydrogen leakage intensity result based on the determined value of leakage intensity or generating a leaked hydrogen quantity result based on the determined value of hydrogen quantity.

By this implementation, not only a mere presence detection, but also a quantitative detection of hydrogen can be provided, due to a strong correlation between the measured temperature and the exothermically converted hydrogen quantities.

According to an advantageous aspect of the invention, the method comprises the steps of:
- determining a value of hydrogen concentration in the gas volume based on a predetermined relation or on predetermined correlated values between at least two of the detected temperature, the detected gas pressure (Pg), a predetermined volume capacity or a predetermined flow rate of the gas volume, and a corresponding hydrogen concentration; and
- generating a hydrogen concentration result based on the determined value of hydrogen concentration.

By this implementation, also a relative detection of hydrogen can be provided under consideration of a closed volume or a flowing gas volume. The concentration is a crucial parameter for the assessment of a risk of explosive gas accumulation.

According to an advantageous aspect of the invention, an optional step of detecting a gas pressure in the gas volume is provided by means of an additional gas pressure sensor, preferably arranged upstream from the catalytic material. By additionally considering the gas pressure which has impact on the dynamics and mobility of hydrogen species contained in the gas volume to be detected, enhanced detection results are obtained due to a more precise determination approach based on more parameters.

According to an advantageous aspect of the invention, the method comprises the step of:
- tempering the catalytic converter material on a predetermined activation temperature associated with a catalytic active state for activation of oxidation reaction of hydrogen.

Depending on the kind of catalytic converter material used, a reaction activity can already be provided at room temperature an above. In case of restrictions of a lower thermal limit of reaction activity or in case of a relatively cold gas or gas flow, the reaction activity of the catalytic converter material can be ensured from the start of operation by an additional tempering by means of a heat source like an electric heat element arranged to the catalytic converter material.

According to advantageous aspects of the invention, the technique is applied to a fuel cell system by exposing and detecting the temperature of the catalytic converter material in a cathode exhaust gas stream, or the technique is applied to an electrolyser cell system by exposing and detecting the temperature of the catalytic converter material in an anode product gas stream. In this way, also an occurrence of leakage not only from an inside to an outside of an electrochemical cell system, but also an occurrence of leakage from a hydrogen containing piping or from a channel within a cell stack into another oxygen containing piping or channel can be detected.

According to an advantageous aspect of the invention, the catalytic converter material is arranged on a gas permeable grid, mesh, channeled or open porous structure, preferably a grid substrate. This embodiment enhances the detectability of a gas flow while providing a flow design of exposure that minimizes a pressure drop in the inspected gas flow.

According to an advantageous aspect of the invention, the catalytic converter material contains a nano structured Platinum metal oxide, preferably in the form of a coating applied onto a titanium grid substrate. This material offers best performance in a substantially linear correlation between the thermal behavior of a rising temperature compared to the converted quantity of hydrogen, as well as an active status at a low temperature level around room temperature.

According to an advantageous aspect of the invention, a test station for testing an electrochemical cell system consuming or producing hydrogen in an electrochemical power conversion, comprises the detection device for detecting a potential hydrogen leakage from the electrochemical cell system into an environment; and a housing surrounding a test space for accommodating the electrochemical cell system; wherein the catalytic converter material of the detection device is exposed to a closed gas volume enclosing the electrochemical cell system or is exposed to a flowing gas volume passing the electrochemical cell system in the housing. In this application, the advantages of a fast response and a simultaneous elimination of potentially explosive hydrogen accumulation are mutually utilized to obtain more precise test results towards leakage inspection at a safer operation, or to obtain lower setup costs upon omitting a dedicated catalysator for explosion prevention.

Further objectives, advantages, features and applications of the present invention arise from the following description of the exemplary embodiments with reference to the drawings. In the drawing:
- Fig. 1: shows a schematic representation of the detection device according to an embodiment of the invention;
- Fig. 2: shows a schematic association of a measured catalyst temperature and correlated values of different detection results in alternative detection domains;
- Fig. 3: shows a schematic representation of a test station according to an embodiment of the invention with the detection device arranged in a closed gas volume; and
- Fig. 4: shows a schematic representation of the test station according to another embodiment of the invention with the detection device arranged in a gas volume of a venting gas flow.

### Detailed description of the Invention

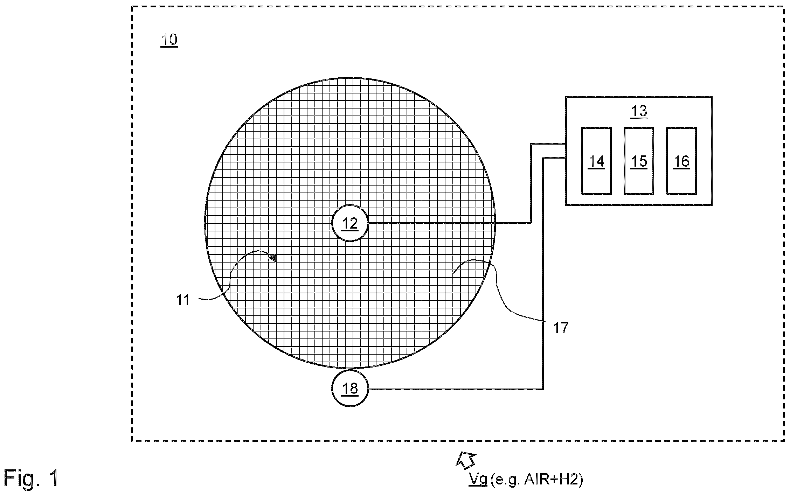

Fig. 1 shows a block diagram of essential parts and functional modules of a detection device 10 for detecting hydrogen in a gas volume Vg which might contain, in most cases, air and a potential unknown amount of hydrogen, as e.g. in the case of a potential leakage from a leakage source.

The detection device 10 comprises a hydrogen oxidizing catalytic converter material 11, e.g. a platinum-based metal oxide or at least platinum alloy containing metal oxide having the capability of activating, under the presence of oxygen, an oxidation reaction of hydrogen to water at comparable low activation temperature Tactive, preferably at approximately room temperature. That means that any hydrogen species spread within the considered gas volume Vg which might be contained in a closed space capacity or might be moving in a gas stream of a certain flow rate, and touching a surface of the exposed catalytic converter material 11 at at least the activation temperature Tactive or higher, will be catalytically converted into water while setting free an exothermic amount of heat originating from an enthalpy of the oxidation reaction.

In the depicted embodiment, the detection device 10 is designed to detect hydrogen from a moving gas volume Vg flowing in a gas stream. In this purpose, the detection device 10 has two opposite openings (not depicted) for entering, guiding and exiting a gas flow. The catalytic converter material 11 is applied onto substrate structure 17 in the form of a coating. The substrate structure 17 is gas flow permeable and can comprise e.g. at least one or several stacked screens or layers of a wire mesh or a grid, or alternatively an open-porous or longitudinal channeled substrate element arranged across a flow diameter in the detection device 10. For a reason of achieving a low pressure drop in a gas flow, the substrate structure 17 in the present embodiment is built of some stacked layers of a titanium grid coated with the catalytic converter material 11.

In an alternative embodiment, the detection device 10 has no flow design for guiding a gas glow through the catalytic converter material 11, but rather an open chamber or just a base plate for mounting the essential parts of the detection device 10 together so that the catalytic converter material 11 is left open exposed to a space of the considered gas volume Vg.

A temperature sensor 12 is arranged in contact with the catalytic converter material 11. Alternatively, the temperature sensor 12 is arranged on a downstream side close to the catalytic converter material 11 in a flow design of the detection device 10. The temperature sensor 12 thus detects a temperature Tcc directly or indirectly related to the catalytic converter material 11. That means in the direct measurement, measured temperature values of the detected temperature Tcc directly render a thermal impact of exothermic reaction activity on the surface of the catalytic converter material 11. In the indirect measurement downstream close to the catalytic converter material 11, measured temperature values of the detected temperature Tcc indirectly render the thermal impact of exothermic reaction activity into a gas flow by a rise in temperature of the gas flow being heated between entering and exiting the detection device 10 when passing the reaction activities at the catalytic converter material 11.

A signal line of the temperature sensor 12 is connected to a temperature monitoring unit 13. The temperature monitoring unit 13 is realized as a microcomputer or a central processing unit (CPU) including hardware means like a processor and a memory for processing and storing data of measured values of the detected temperature Tcc and other predetermined correlated values of different physical parameters, and for calculating values based on predetermined relations between the detected temperature Tcc and the other different physical parameters by a software program.

As an optional modification to the embodiment, an additional gas pressure sensor 18 can be arranged in the near of the catalytic converter material 11. In a flow design of the detection device 10, the gas pressure sensor 18 is arranged on an upstream side close to the catalytic converter material 11. The gas pressure sensor 18 thus detects a gas pressure Pg of the gas volume Vg in an area of the catalytic converter material 11. The gas pressure Pg influences how much of the hydrogen species contained in the gas volume Vg will get in contact with, and thus, will exothermically react on the catalytic converter material 11. A signal line of the gas pressure sensor 18 is connected to the temperature monitoring unit 13. Hence, particularly in a flow design of the detection device 10 and under a potential flow rate, the gas pressure Pg of the gas volume Vg can be optionally detected and additionally considered in a determination of the hydrogen detection for enhanced quality of detection results.

The software program implements, on the hardware means of the temperature monitoring unit 13, different functional modules, including a comparator module 14, a generating module 15 and a determination module 16, as explained in the following. A comparator module 14 receives measured values of the temperature Tcc detected by the temperature sensor 12. The comparator module 14 has access to a memory in which at least one predetermined threshold value, preferably several threshold values of an exothermic temperature Texo, or alternatively, values of an exothermic temperature range TRexo are stored. The stored predetermined exothermic temperature Texo or the exothermic temperature range TRexo represent reaction activities of oxidation reaction at the catalytic converter material 11 and are higher in value than the activation temperature Tactive due to the additional exothermic heat impact of the oxidation reaction of hydrogen. The comparator module 14 compares the received values of the detected temperature Tcc with the stored exothermic temperature Texo and determines, whether the current value of the detected temperature Tcc is lower, higher or equal to the threshold value of the exothermic temperature Texo. Alternatively, the comparator module 14 compares the received values of the detected temperature Tcc with the stored exothermic temperature range TRexo and determines, whether the current value of the detected temperature Tcc falls within the exothermic temperature range TRexo. In case the detected temperature Tcc is equal or higher than the exothermic temperature Texo, or the detected temperature Tcc falls within the exothermic temperature range TRexo, the comparator module 14 deposits a positive determination.

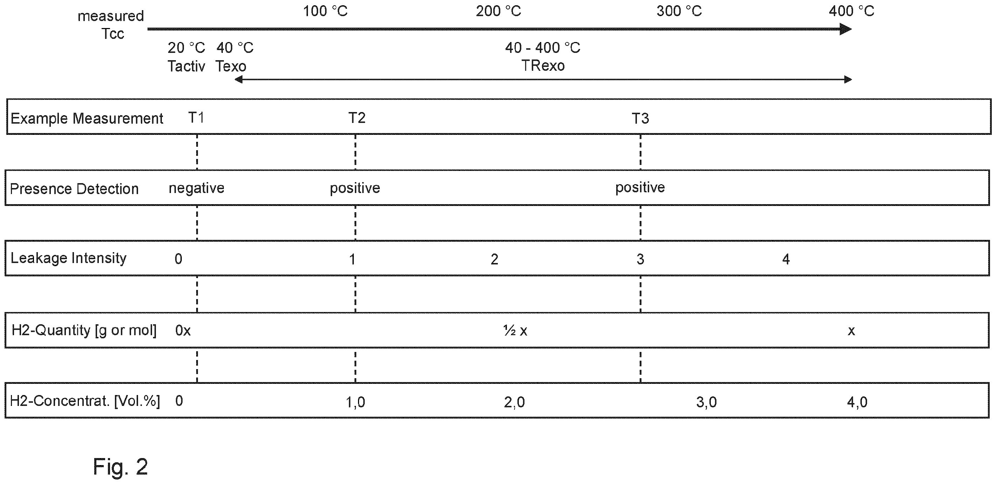

In case of a preceding positive determination deposited by the comparator module 14, the generating module 15 generates a positive hydrogen detection result indicating a presence of hydrogen in the gas volume Vg, as depicted in Fig. 2 for exemplified measured values T2 and T3 of the detected temperature Tcc. Otherwise, the generating module 15 generates a negative hydrogen detection result indicating that no hydrogen is present in the gas volume Vg, as depicted in Fig. 2 for exemplified measured value T1 of detected temperature Tcc. Depending on a configuration, the generating module 15 does not only generate the detection result but may also output a notification including the detection result to a user, or may output a signal to a control of a hydrogen conducting system for triggering an operational relevant function, such as a shutdown function.

Furthermore, the generating module 15 can be configured to generate different further domains of detection result depending on a preceding domain of determination of the determination module 16, as explained in the following.

The determination module 16 has access to a calculation capacity of the processor and to a memory having stored therein formulars including relations between different physical parameters including the detected temperature Tcc and optionally the detected gas pressure Pg, or alternatively, a look-up table of experimentally predetermined correlating values of the different physical parameters including the detected temperature Tcc and optionally the detected gas pressure Pg.

In different implementation examples, the determination module 16 can determine different domains of detection results and cause the generating module 15 to generate a detection result corresponding to one of the different detection result domains provided. Those different domains of detection result include a positive or negative hydrogen presence result as explained before, a leakage intensity result, a hydrogen quantity result, or a hydrogen concentration result.

In one implementation example regarding the detection domain of leakage intensity, the determination module 16 enters a predetermined formular based on a relation between the detected temperature Tcc and a predetermined value or a normalized level of a leakage intensity, e.g. level 0 to level 4. In this case, the calculation of the hydrogen quantity involves a predetermined factor expressing a linear or non-linear correlation between the detected temperature Tcc and a normalized level of leakage intensity. Upon calculation, the generating module 15 generates an output of the leakage intensity result, e.g. level 0 for T1, level 1 for T2 and level 3 for T3, as depicted in Fig. 2. Alternatively, the leakage intensity can be determined with respect to a leakage flow rate in terms of a volume per time, e.g. liter per hour.

In another implementation example regarding the detection domain of hydrogen quantity, the determination module 16 enters a predetermined formular based on a relation between the detected temperature Tcc and optionally the detected gas pressure Pg, and a predetermined value of a hydrogen quantity with respect to an absolute mass or a molar mass. In this case, the calculation of the hydrogen quantity involves a predetermined factor expressing a linear or non-linear correlation between the detected temperature Tcc and the corresponding hydrogen quantity. Upon calculation, the generating module 15 generates an output of the hydrogen quantity result, e.g. approximately 0,1x g or mol for T1, 0,25x g or mol for T2 and 0,7x g or mol for T3, as depicted in Fig. 2.

In a further implementation example regarding the detection domain of hydrogen concentration, the determination module 16 enters a predetermined formular based on a relation between the detected temperature Tcc and optionally the detected gas pressure Pg, and a predetermined value of a hydrogen concentration with respect to a volumetric percentage, involving a given volume capacity or flow rate of the gas volume Vg containing the hydrogen concentration. In this case, the calculation of the hydrogen concentration involves a predetermined factor expressing a linear or non-linear correlation between the detected temperature Tcc and optionally the detected gas pressure Pg, the corresponding hydrogen quantity and the given volume capacity or flow rate. Upon calculation, the generating module 15 generates an output of the hydrogen concentration result, e.g. approximately 0,1 Vol.% for T1, 1,0 Vol.% for T2 and 2,6 Vol.% for T3, as depicted in Fig. 2.

In a modification of the explained detection result domains regarding the leakage intensity, the hydrogen quantity and the hydrogen concentration, the determination module 16 does not enter a formular, but does enters a look-up table of experimentally predetermined pairs or sets of correlating values of said different physical parameters. Thus, the look-up table provides for each measured value T1, T2, T3 of the detected temperature Tcc as an entry parameter, the same values of detection results in the respective detection result domain as an exit parameter.

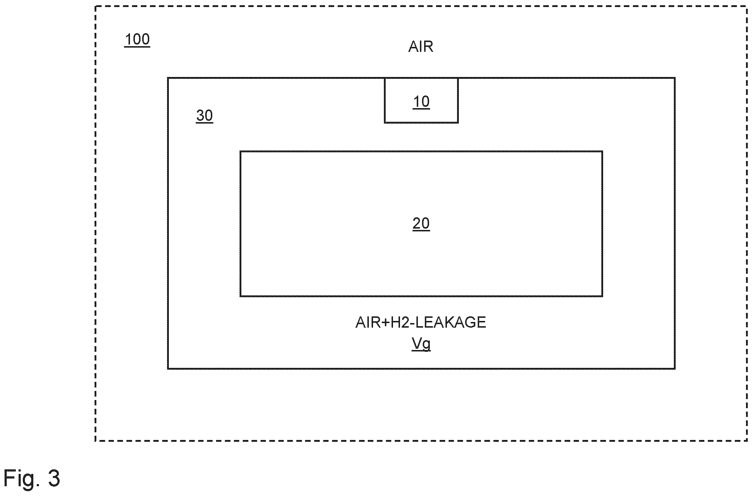

Fig. 3 illustrates a schematic block diagram of a test station 100 for testing any operational aspects of an electrochemical cell system 20, especially a fuel cell system or an electrolyser cell system. These systems include a piping and sealed channels in cell stacks for a strictly separate guidance of two process gases, one of them containing a high hydrogen concentration. In terms of safety tests or wear tests, it is crucial to check, whether all parts for guiding the hydrogen containing process gas stream stay sufficiently sealed to the outside and to other internal parts of the system. For said purpose, the test station 100 allows for surveillance of an atmosphere surrounding the system with all or most of its components.

The test station 100 comprises a housing 30 enclosing a test space for accommodating the electrochemical cell system 20. The remaining volume within the test space that is not occupied by the electrochemical cell system 20 represents the gas volume Vg filled with air and a potentially leaked hydrogen content. Outside of the housing 30 the test station is open to the environment, i.e. a normal provision of air and atmospheric pressure. A detection device 10 in line with an embodiment of the before mentioned implementation examples is arrange inside the housing 30 to continuously inspect the gas volume Vg enclosing the electrochemical cell system 20 for possible hydrogen gas leakages.

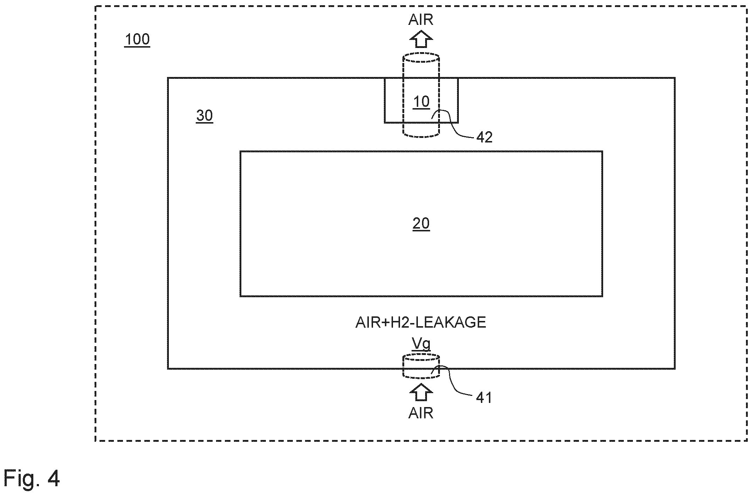

Fig. 4 shows a modification of the embodiment of the test station 100 of Fig. 3. Most parts of the test station 100 in Fig. 4 are identical to the test station 100 in Fig. 3. except for an additional venting system including a venting inlet 41 and a venting outlet 42 as well as a fan (not depicted) for producing a forced venting of the test space at a controlled venting flow rate. Such forced venting further reduces the risk of explosive hydrogen accumulation within the housing 30. Under consideration of the gas flow through the inspected gas volume Vg, it is essential that a formular or a look-up table involves a corresponding value of the flow rate of fresh venting air. Said flow rate can be simultaneously measured, or can be constantly preset and met, or otherwise provided for the respective domain of determination result.

The foregoing explanations of the embodiments describe the present invention solely by way of examples. Of course, individual features of the embodiments can be freely combined with one another, provided this is technically expedient, without departing from the scope of the present invention.

### List of reference characters

- 10: detection device
- 11: catalytic converter material (coating)
- 12: temperature sensor
- 13: temperature monitoring unit
- 14: comparator module
- 15: detection module
- 16: determination module
- 17: grid substrate carrying the coating of catalytic converter material
- 20: electrochemical cell system (potential leakage source)
- 30: housing
- 41: venting inlet
- 42: venting outlet
- 100: test station

- Pg: gas pressure detected in the gas volume
- T1: first example measurement of Tcc
- T2: second example measurement of Tcc
- T3: third example measurement of Tcc
- Tactive: activation temperature
- Tcc: measured temperature at catalytic converter
- Texo: exothermic temperature
- TRexoexothermic: temperature range
- Vg: gas volume

## Claims

1. Method for detecting hydrogen from a gas volume (Vg), including the step of:
- exposing a hydrogen oxidizing catalytic converter material (11) to the gas volume (Vg);
**characterized by** the steps of:
- detecting a temperature (Tcc) of the catalytic converter material (11);
- comparing the detected temperature (Tcc) with at least one predetermined exothermic temperature (Texo) associated with a thermal impact of exothermic heat from oxidation reaction activities at the catalytic converter material (11); and
- generating a hydrogen detection result indicating at least a presence of hydrogen in the gas volume (Vg), if the detected temperature (Tcc) is equal or higher than the at least one exothermic temperature (Texo).

2. Method according to claim 1, comprising the steps of:
- determining a value of leakage intensity or a value of hydrogen quantity of hydrogen in the gas volume (Vg) based on a predetermined relation or based on predetermined correlated values between at least one of the detected temperature (Tcc) and a detected gas pressure (Pg), and a corresponding leakage intensity or a corresponding hydrogen quantity; and
- generating a hydrogen leakage intensity result based on the determined value of leakage intensity or generating a leaked hydrogen quantity result based on the determined value of hydrogen quantity.

3. Method according to claim 1 or 2, comprising the steps of:
- determining a value of hydrogen concentration in the gas volume (Vg) based on a predetermined relation or based on predetermined correlated values between at least two of the detected temperature (Tcc), the detected gas pressure (Pg), a predetermined capacity or a predetermined flow rate of the gas volume (Vg), and a corresponding hydrogen concentration; and
- generating a hydrogen concentration result based on the determined value of hydrogen concentration.

4. Method according to one of the preceding claims, comprising the step of:
- tempering the catalytic converter material (11) on a predetermined activation temperature (Tactiv) associated with a catalytic active state for activating oxidation reaction of hydrogen.

5. Method according to one of the preceding claims, wherein at least the steps of:
- exposing the hydrogen oxidizing catalytic converter material (11) to the gas volume (Vg); and
- detecting the temperature (Tcc) of the catalytic converter material (11);
are conducted in a cathode exhaust gas stream of a fuel cell.

6. Method according to one of the preceding claims, wherein at least the steps of:
- exposing the hydrogen oxidizing catalytic converter material (11) to the gas volume (Vg); and
- detecting the temperature (Tcc) of the catalytic converter material (11);
are conducted in an anode product gas stream of an electrolyser cell.

7. Detection device (10) for detecting hydrogen from a gas volume (Vg), comprising:
a hydrogen oxidizing catalytic converter material (11) arranged exposed to the gas volume (Vg);
**characterized in that**
the detection device (10) comprises:
a temperature sensor (12) arranged to detect a temperature (Tcc) of the catalytic converter material (11); and
a temperature monitoring unit (13) for monitoring the detected temperature (Tcc), including the following functional modules:
a comparator module (14) configured to compare the detected temperature (Tcc) with at least one predetermined exothermic temperature (Texo) associated with a thermal impact of exothermic heat from oxidation reaction activities at the catalytic converter material (11), and
a generating module (15) configured to generate a hydrogen detection result indicating at least a presence of hydrogen in the gas volume (Vg), if the detected temperature (Tcc) is equal or higher than the at least one exothermic temperature (Texo).

8. Detection device (10) according to claim 7, comprising:
a gas pressure sensor (18) for detecting a gas pressure (Pg) in the gas volume (Vg), preferably arranged upstream to the catalytic converter material (11).

9. Detection device (10) according to claim 7 or 8, wherein the temperature monitoring unit (13) includes the following functional module:
a determination module (16) configured to determine a value of leakage intensity or to determine a value of hydrogen quantity in the gas volume (Vg) based on a predetermined relation or based on predetermined correlated values between at least one of the detected temperature (Tcc) and the detected gas pressure (Pg), and a corresponding leakage intensity or a corresponding hydrogen quantity; wherein
the generating module (15) is further configured to generate a hydrogen leakage intensity result based on the determined value of leakage intensity, or to generate a hydrogen quantity result based on the determined value of hydrogen quantity.

10. Detection device (10) according to any of claims 7 to 9, wherein the temperature monitoring unit (13) includes the following functional module:
the determination module (16) further configured to determine a value of hydrogen concentration in the gas volume (Vg) based on a predetermined relation or based on predetermined correlated values between at least two of the detected temperature (Tcc), the detected gas pressure (Pg), a predetermined capacity or a predetermined flow rate of the gas volume (Vg), and a corresponding hydrogen concentration; wherein
the generating module (15) is further configured to generate a hydrogen concentration result based on the determined value of hydrogen concentration.

11. Detection device (10) according to any of claims 7 to 10, comprising:
a heat source, preferably an electrical heating element, arranged at the catalytic converter material (11), for tempering the catalytic converter material (11) on a predetermined activation temperature (Tactiv) associated with a catalytic active state for activating oxidation reaction of hydrogen.

12. Detection device (10) according to any of claims 7 to 11, wherein the catalytic converter material (11) is arranged on a gas permeable grid, mesh, channeled or open porous structure, preferably a grid substrate (17), wherein the catalytic converter material (11) especially contains a nano structured Platinum metal oxide, preferably in the form of a coating applied onto a titanium grid substrate (17).

13. Fuel cell system for generating electric power from a hydrogen containing fuel gas and an oxygen containing oxidation gas by means of at least one fuel cell, comprising:
the detection device (10) according to any of claims 7 to 13 for detecting a potential hydrogen leakage in the fuel cell system, wherein
the catalytic converter material (11) of the detection device (10) is exposed to a gas volume (Vg) flowing in a cathode exhaust gas stream of the at least one fuel cell.

14. Electrolyser system for generating a hydrogen containing product gas and an oxygen containing product gas from water and electric power by means of at least one electrolyser cell, comprising:
the detection device (10) according to any of claims 7 to 13 for detecting a potential hydrogen leakage in the electrolyser cell system, wherein
the catalytic converter material (11) of the detection device (10) is exposed to a gas volume (Vg) flowing in an anode product gas stream of the at least one electrolyser cell.

15. Test station (100) for testing an electrochemical cell system (20) consuming or producing hydrogen in an electrochemical power conversion, comprising:
the detection device (10) according to any of claims 7 to 13 for detecting a potential hydrogen leakage from the electrochemical cell system (20) into an environment; and
a housing (30) surrounding a test space for accommodating the electrochemical cell system (20); wherein
the catalytic converter material (11) of the detection device (10) is exposed to a closed gas volume (Vg) enclosing the electrochemical cell system (20) or is exposed to a flowing gas volume (Vg) passing the electrochemical cell system (20) in the housing (30).
